# EUROPÄISCHE PATENTSCHRIFT

(11) **EP 3 818 294 B1**
(45) Veröffentlichungstag und Bekanntmachung des Hinweises auf die Patenterteilung: **25.10.2023**
(21) Anmeldenummer: 19728932.5
(22) Anmeldetag: 03.06.2019
(51) Int. Cl.: F16N 29/00, G01N 33/28, G01N 1/20

(54) **BEREITHALTUNG EINER VALIDEN SCHMIERSTOFFPROBE**
CONTINUOUS PROVISION OF A VALID LUBRICANT SAMPLE
MISE À DISPOSITION D'UN ÉCHANTILLON DE LUBRIFIANT VALIDE

(30) Priorität: 06.07.2018 DE 102018211164; 29.11.2018 DE 102018220584
(43) Veröffentlichungstag der Anmeldung: 12.05.2021
(73) Patentinhaber: ZF Friedrichshafen AG, 88046 Friedrichshafen (DE)
(72) Erfinder: VATH, Andreas, 63849 Leidersbach (DE); BERGER, Günter, 44577 Castrop-Rauxel (DE); TENCKHOFF, Georg, 88048 Friedrichshafen (DE)
(74) Vertreter: ZF Friedrichshafen AG
(86) Internationale Anmeldenummer: PCT/EP2019/064283
(87) Internationale Veröffentlichungsnummer: WO 2020/007545

(56) Entgegenhaltungen:
- WO-A1-00/32980
- WO-A1-2018/085875
- DE-A1-102010 004 917
- DE-C- 374 396
- GB-A- 1 501 903
- US-A- 4 649 711

## Beschreibung

Die Erfindung betrifft eine Anordnung nach dem Oberbegriff von Anspruch 1 und ein Verfahren nach Anspruch 10.

Bei vielen technischen Systemen ist es notwendig, in regelmäßigen Zeitabständen den Zustand des Schmiermittels zu überprüfen. Dazu werden Ölproben entnommen. Damit diese Aussagekraft über den Zustand des Systems haben, müssen sie einem durchströmten Bereich entnommen werden, wenn das System seine Betriebstemperatur erreicht hat. Eine solche Ölprobe wird valide genannt.

Die Entnahme von Ölproben ist häufig beschränkt. Gründe sind etwa schlechte Zugänglichkeit oder Sicherheitsaspekte. Ein Beispiel für ein schlecht zugängliches technisches System ist eine Windkraftanlage. Um hier eine Ölprobe zu entnehmen, muss ein Monteur den Turm der Windkraftanlage besteigen und manuell das Getriebe öffnen.

Die Druckschrift WO 2017/062273 A1 offenbart einen Behälter, der sich über ein schaltbares Ventil mit Schmierstoff befüllen lässt. Mittels des Behälters lässt sich eine Schmierstoffprobe gewinnen. Schmierstoff, mit dem der Behälter befüllt wurde, verbleibt in dem Behälter, bis der Behälter manuell entnommen wurde. Ohne manuelle Entnahme ist es nicht möglich, den Behälter erneut zu befüllen.

Die WO 2018/085875 A1 offenbart eine Anordnung von Mitteln, die einen Schmierstoffkreislauf ausbilden, nach dem Oberbegriff der Ansprüche 1,2 und ein Verfahren zur Bereithaltung einer validen Schmierstoffprobe nach dem Oberbegriff des Anspruchs 7.

Der Erfindung liegt die Aufgabe zugrunde, die Bereitstellung einer validen Ölprobe zu verbessern. Insbesondere soll die Bereitstellung flexibler erfolgen und die Entnahme vereinfacht werden.

Diese Aufgabe wird gelöst durch eine Anordnung nach Anspruch 1 und ein Verfahren nach Anspruch 10. Bevorzugte Weiterbildungen sind in den Unteransprüchen enthalten.

Die Anordnung weist Mittel auf, die einen Schmierstoffkreislauf ausbilden. Zu den Mitteln gehören eine erste Schmierstoffleitung, mindestens eine Leitungsverzweigung und mindestens eine Leitungszusammenführung. Die Leitungsverzweigung verzweigt die erste Schmierstoffleitung. Durch die Verzweigung entstehen ein erster Abzweig und ein zweiter Abzweig. Von der ersten Schmierstoffleitung geleiteter Schmierstoff wird in der Leitungsverzweigung aufgeteilt in einen ersten Teil und einen zweiten Teil. Der erste Teil des Schmierstoffs fließt durch den ersten Abzweig, der zweite Teil durch den zweiten Abzweig.

Die Leitungsverzweigung kann etwa als Knotenpunkt mit einem ersten Anschluss, einem zweiten Anschluss und einem dritten Anschluss ausgebildet sein. Der erste Anschluss, der zweite Anschluss und der dritte Anschluss sind schmierstoffleitend miteinander verbunden. Weiterhin ist die Schmierstoffleitung schmierstoffleitend mit dem ersten Anschluss verbunden; der erste Abzweig ist schmierstoffleitend mit dem zweiten Anschluss verbunden, und der zweite Abzweig ist schmierstoffleitend mit dem dritten Anschluss verbunden.

Die Leitungszusammenführung führt den ersten Teil des Schmierstoffs und den zweiten Teil des Schmierstoffs zusammen. Durch die Zusammenführung mischen sich also der erste Teil des Schmierstoffs und der zweite Teil des Schmierstoffs. Die Leitungszusammenführung kann analog zu der Leitungsverzweigung als ein Knotenpunkt mit einem ersten Anschluss, einem zweiten Anschluss und einem dritten Anschluss ausgestaltet sein. Der erste Anschluss, der zweite Anschluss und der dritte Anschluss sind schmierstoffleitend miteinander verbunden. Schmierstoff leitend mit dem ersten Anschluss ist weiterhin der erste Abzweig verbunden. Der zweite Abzweig ist schmierstoffleitend mit dem zweiten Anschluss verbunden. Der dritte Anschluss mündet in eine zweite Schmierstoffleitung und verbindet diese schmierstoffleitend.

Erfindungsgemäß ist mindestens ein Schmierstoffspeicher vorgesehen, der in den ersten Abzweig integriert ist. Entsprechend durchfließt der erste Teil des Schmierstoffs, der den ersten Abzweig durchfließt, auch den Schmierstoffspeicher. Die Integration des Schmierstoffspeichers in den ersten Abschnitt erfolgt derart, dass der Schmierstoffspeicher einen Schmierstoffzufluss und einen Schmierstoffabfluss aufweist, die schmierstoffleitend in den ersten Abschnitt integriert sind. Durch den Schmierstoffzufluss fließt der erste Teil des Schmierstoffs in den Schmierstoffspeicher hinein. Durch den Schmierstoffabfluss fließt der erste Teil des Schmierstoffs aus dem Schmierstoffspeicher hinaus. Der in den Schmierstoffspeicher hineingeflossene und in dem Schmierstoffspeicher befindliche Schmierstoff steht als Schmierstoffprobe zur Verfügung.

Um die Befüllung des Schmierstoffspeichers zu steuern, ist der erste Abzweig sperr- und freigebbar. Im gesperrten Zustand ist der erste Abzweig schmierstoffdicht verschlossen. Sämtlicher Schmierstoff fließt dann von der ersten Leitung über die Leitungsverzweigung durch den zweiten Abzweig, der eine schmierstoffleitende Verbindung zwischen der ersten Schmierstoffleitung und der zweiten Schmierstoffleitung bildet. Insbesondere fließt der Schmierstoff nicht in den Schmierstoffspeicher, wenn der erste Abzweig gesperrt ist.

Im freigegebenen Zustand ist der erste Abzweig schmierstoffleitend. Es besteht dann über den ersten Abzweig eine s schmierstoffleitende Verbindung zwischen der ersten Schmierstoffleitung und der zweiten Schmierstoffleitung. Insbesondere ist der erste Abzweig für den ersten Teil des Schmierstoffs aus der Leitungsverzweigung durchlässig. Dies bedeutet, dass der erste Teil des Schmierstoffs durch den ersten Abzweig fließt, wenn der erste Abzweig freigegeben ist. Der erste Teil des Schmierstoffs fließt dann, wie oben beschrieben, auch durch den Schmierstoffspeicher.

Die Erfindung ermöglicht es, den ersten Abzweig freizugeben, sobald der Schmierstoff einen validen Zustand annimmt. Der Schmierstoff, der dann in den Schmierstoffspeicher gelangt, stellt eine valide Schmierstoffprobe dar und kann entnommen und analysiert werden. Insbesondere ist es möglich, den ersten Abzweig zu sperren, wenn der valide Zustand des Schmierstoffs sich ändert. Trotz der Änderung des Zustands verbleibt dann in dem Schmierstoffspeicher eine Schmierstoffprobe, die aus validem Schmierstoff gewonnen wurde. Die Erfindung ermöglicht es zudem, eine bereits in dem Schmierstoffbehälter befindliche Schmierstoffprobe - sofern diese nicht entnommen werden soll - durch eine neuere Schmierstoffprobe zu ersetzen.

Zum Sperren und Freigeben des ersten Abzweigs ist in einer bevorzugten Weiterbildung mindestens ein Ventil in den ersten Abzweig integriert. Das Ventil ist schließ- und öffenbar. Im geschlossenen Zustand sperrt das Ventil den ersten Abzweig. Im geöffneten Zustand gibt das Ventil entsprechend den ersten Abzweig frei.

Auch kann das Ventil in einer bevorzugten Weiterbildung in die Leitungsverzweigung oder in die Leitungszusammenführung integriert sein. In diesem Fall ist das Ventil bevorzugt als Umschaltventil ausgestaltet. Das Umschaltventil gibt wahlweise den ersten Abzweig frei und sperrt den zweiten Abzweig oder gibt den zweiten Abzweig frei und sperrt den ersten Abzweig.

Das Ventil ist, gemäss der Erfindung, temperaturgesteuert. Dies bedeutet, dass das Ventil in Abhängigkeit einer Temperatur des Schmierstoffs öffnet oder schließt. Das Ventil lässt sich etwa durch Bimetallfedern, durch Federn aus Formgedächtnislegierung, durch ein Dehnstoffelement oder elektromechanisch mittels eines Temperatursensors und eines auf das Ventil wirkenden Aktors temperaturabhängig steuern.

Zusätzlich zu dem in den ersten Abzweig integrierten Ventil ist in einer bevorzugten Weiterbildung ein Ventil in den zweiten Abzweig integriert. Das in den ersten Abzweig integrierte Ventil und das in den zweiten Abzweig integrierte Ventil öffnen und schließen vorzugsweise wechselseitig. Dies bedeutet, dass das in den zweiten Abzweig integrierte Ventil geschlossen ist, wenn das in den ersten Abzweig integrierte Ventil geöffnet ist. Umgekehrt ist das in den zweiten Abzweig integrierte Ventil geöffnet, wenn das in dem ersten Abzweig integrierte Ventil geschlossen ist. Auf diese Weise lässt sich der von der ersten Schmierstoffleitung geleitete Schmierstoff vollständig wahlweise durch den ersten Abzweig oder den zweiten Abzweig leiten.

Ist in dem zweiten Abzweig kein Ventil vorgesehen, fließt der Schmierstoff vollständig durch den zweiten Abzweig, wenn das in den ersten Abzweig integrierte Ventil geschlossen ist. Ist dieses aber geöffnet, fließt ein Teil des Schmierstoffs weiterhin durch den zweiten Abzweig.

Um den Schmierstoff durch den Schmierstoffkreislauf zu fördern, ist in einer darüber hinaus bevorzugten Weiterbildung eine in den Schmierstoffkreislauf integrierte Pumpe vorgesehen. Diese ist bezüglich eines durch den Schmierstoffkreislauf verlaufenden Schmierstoffflusses vor der Leitungsverzweigung oder hinter der Leitungszusammenführung angeordnet. Dies bedeutet, dass der Schmierstoff im erstgenannten Fall von der Pumpe zu der Leitungsverzweigung fließt. Im letztgenannten Fall fließt der Schmierstoff von der Leitungszusammenführung zu der Pumpe.

Anstelle von Ventilen lassen sich auch Pumpen zur Steuerung des Schmierstoffflusses einsetzen. In einer bevorzugten Weiterbildung umfasst die Anordnung entsprechend eine erste Pumpe und eine zweite Pumpe. Die erste Pumpe ist in den ersten Abzweig integriert, die zweite Pumpe in den zweiten Abzweig. Indem wahlweise die erste Pumpe eingeschaltet und die zweite Pumpe ausgeschaltet oder die zweite Pumpe eingeschaltet und die erste Pumpe ausgeschaltet wird, lässt sich der Schmierstofffluss steuern. Im erstgenannten Fall fließt der Schmierstoff durch den ersten Abzweig, im letztgenannten Fall durch den zweiten Abzweig. Auch ist es möglich, beide Pumpen zugleich einzuschalten. In diesem Fall verzweigt sich der Schmierstoff zu jeweils einem Teil auf beide Abzweige.

In einer darüber hinaus bevorzugten Weiterbildung ist mindestens ein Rückschlagventil in den ersten Abzweig integriert. Das Rückschlagventil verhindert, dass der Schmierstoffspeicher leerläuft. Dazu ist das Rückschlagventil bezüglich eines durch den ersten Abzweig verlaufenden Schmierstoffflusses zwischen der Leitungsverzweigung und dem Schmierstoffspeicher angeordnet. Dies bedeutet, dass Schmierstoff, der von der Leitungsverzweigung in den Schmierstoffspeicher fließt, das Rückschlagventil passiert, bevor er in den Schmierstoffspeicher gelangt. Das Rückschlagventil ist so ausgerichtet, dass es den passierenden Schmierstoff durchlässt. In Gegenrichtung sperrt es.

Die Anordnung weist erfindungsgemäss mindestens einen Schmierstofffilter auf.

Dieser ist hinter dem Schmierstoffspeicher in den Schmierstoffkreislauf eingebunden. Bevorzugt ist der Schmierstofffilter bezüglich des durch den Schmierstoffkreislauf verlaufenden Schmierstoffflusses hinter der Leitungszusammenführung angeordnet. Dies bedeutet, dass Schmierstoff, der den Schmierstofffilter passiert, zuvor durch die Leitungszusammenführung geflossen ist.

Ein erfindungsgemäßes Verfahren verwendet die erfindungsgemäße Anordnung oder eine bevorzugte Weiterbildung zur Bereithaltung einer validen Schmierstoffprobe. Das Verfahren sieht vor, eine oder mehrere physikalische Größen des in dem Schmierstoffkreislauf zirkulierenden Schmierstoffs zu messen.

Bei einer physikalischen Größe handelt es sich um eine quantitativ bestimmbare Eigenschaft eines physikalischen Objekts, im vorliegenden Fall des Schmierstoffs. Als zu messende physikalische Größen kommen etwa die Temperatur, die Strömungsgeschwindigkeit, der Volumenstrom, die Feuchte bzw. der Wassergehalt des Schmierstoffs und/oder die seit dem letzten Einschalten bzw. der letzten Inbetriebnahme der Anordnung verstrichene Zeit infrage.

Das erfindungsgemäße Verfahren sieht vor, das in den ersten Abzweig integrierte Ventil, das als Umschaltventil ausgebildete Ventil, das in den zweiten Abzweig integrierte Ventil, die erste Pumpe und/oder die zweite Pumpe in Abhängigkeit der gemessenen physikalischen Größen zu steuern. Dadurch lässt sich in Abhängigkeit der gemessenen physikalischen Größen gezielt die Verteilung des Schmierstoffs auf den ersten Abzweig und den zweiten Abzweig beeinflussen. Dadurch wiederum wird die Befüllung des Schmierstoffspeichers gesteuert.

Die Anordnung weist in einer bevorzugten Weiterbildung mindestens ein Steuergerät auf, das ausgebildet ist, das erfindungsgemäße Verfahren auszuführen.

Bevorzugte Ausführungsbeispiele der Erfindung sind in den Figuren dargestellt. Übereinstimmende Bezugsziffern kennzeichnen dabei gleiche oder funktionsgleiche Merkmale. Im Einzelnen zeigt:
Fig. 1 einen ersten Schmierstoffkreislauf; und
Fig. 2 einen zweiten Schmierstoffkreislauf.

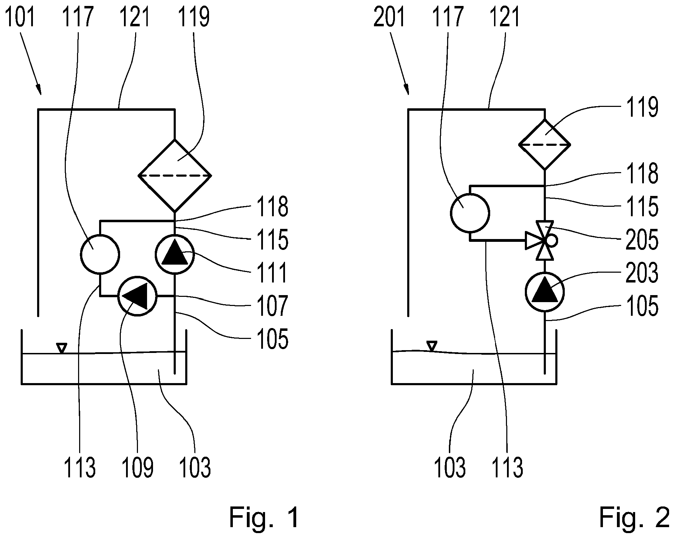

Der in Fig. 1 dargestellte Schmierstoffkreislauf 101 umfasst einen Ölsumpf 103, eine erste Schmierstoffleitung 105, eine Leitungsverzweigung 107, eine erste Pumpe 109, eine zweite Pumpe 111, einen ersten Abzweig 113, einen zweiten Abzweig 115, einen Probenbehälter 117, einen Ölfilter 119 und eine zweite Leitung 121.

Das Öl in dem Schmierstoffkreislauf 101 wird mittels der ersten Pumpe 109, der zweiten Pumpe 111 oder beider Pumpen 109, 111 zusammen gefördert. Die erste Pumpe 109 befindet sich in dem ersten Abzweig 113, die zweite Pumpe 111 in dem zweiten Abzweig 115. Die Leitungsverzweigung 107 verbindet den ersten Abzweig 113, den zweiten Abzweig 115 und die erste Leitung 105 schmierstoffleitend miteinander.

Die erste Leitung 105 ragt in den Schmierstoffsumpf 103 hinein. Auf diese Weise können die Pumpen 109, 111 Öl aus dem Schmierstoffsumpf 103 ansaugen. Die Leitungszusammenführung 115 leitet das Öl, das durch den ersten Abzweig 113 und/oder den zweiten Abzweig 105 fließt, in die zweite Leitung 121 ein. Dort fließt es durch den in der zweiten Leitung 121 angeordneten Ölfilter 119. Die zweite Leitung 121 transportiert das Öl zu Schmierstellen, etwa Lager oder Zahnräder. Von dort aus fließt das Öl zurück in den Schmierstoffsumpf 103.

Nach dem Einschalten des Schmierstoffkreislaufs 101 ist es aufgrund der zu geringen Temperatur des Öls zunächst noch nicht möglich, eine valide Ölprobe zu entnehmen. Bis das Öl eine ausreichende Temperatur erreicht hat, fördert die zweite Pumpe 111 das Öl. Die zweite Pumpe 109 kann bei Bedarf zugeschaltet werden, etwa um die Förderleistung zu erhöhen.

Sobald das Öl so warm geworden ist, dass eine valide Ölprobe entnommen werden kann, wird die erste Pumpe 109 - sofern noch nicht geschehen - zugeschaltet. Je nach Dimensionierung der ersten Pumpe 109 und des ersten Abzweigs 113 kann die erste Pumpe 111 dann ausgestaltet werden oder weiterlaufen. Die erste Pumpe 109 fördert - wenn die zweite Pumpe 111 weiterläuft - einen Teil des Öls oder - wenn die zweite Pumpe 111 abgeschaltet wird - das Öl vollständig von der Leitungsverzweigung 107 über den ersten Abzweig 113 zu der Leitungszusammenführung 115. Da das Probenbehältnis 117 in den ersten Abzweig 113 integriert ist, füllt es sich nun mit Öl, das eine valide Ölprobe darstellt.

Anschließend wird die erste Pumpe 109 ausgeschaltet. Das Öl fließt nun von dem Ölsumpf 103 durch die erste Leitung 105 und die Leitungsverzweigung 107 vollständig über den zweiten Abzweig 105. Da kein Öl über den ersten Abzweig 113 fließt, gelangt kein Öl in das Probenbehältnis 117, sodass die Ölprobe valide bleibt.

Der in Fig. 2 dargestellte Schmierstoffkreislauf 201 unterscheidet sich von dem Schmierstoffkreislauf 101 aus Fig. 1 dadurch, dass nur eine einzige Pumpe 203 anstelle der ersten Pumpe 109 und der zweiten Pumpe 111 vorgesehen ist. Die Pumpe 203 befindet sich in der ersten Leitung 105 und fördert über die erste Leitung 105 Öl aus dem Ölsumpf 103 zu dem Umschaltventil 205.

Zudem ersetzt ein Umschaltventil 205 die Leitungsverzweigung 107. Wie die Leitungsverzweigung 107 weist das Umschaltventil 205 einen ersten Anschluss, einen zweiten Anschluss und einen dritten Anschluss auf. Die erste Leitung 105 ist schmierstoffleitend mit dem ersten Anschluss verbunden. Der zweite Anschluss ist schmierstoffleitend mit dem ersten Abzweig 113 verbunden, der dritte Anschluss mit dem zweiten Abzweig 215.

Innerhalb des Umschaltventils 205 besteht eine schmierstoffleitende Verbindung zwischen dem ersten Anschluss, dem zweiten Anschluss und dem dritten Anschluss. Diese Verbindung ist allerdings nicht statisch, sondern kann durch Betätigung des Umschaltventils 205 variiert werden.

Befindet sich das Umschaltventil 205 in einer ersten Stellung, sind der erste Anschluss und der zweite Anschluss und damit die erste Leitung 105 und der erste Abzweig 113 schmierstoffleitend miteinander verbunden. Der dritte Anschluss und damit der zweite Abzweig 115 sind dabei schmierstoffdicht verschlossen. In einer zweiten Position des Umschaltventils 205 sind der zweite Anschluss und damit der erste Abzweig 113 schmierstoffdicht verschlossen, während eine schmierstoffleitende Verbindung zwischen dem ersten Anschluss und dem dritten Anschluss und damit zwischen der ersten Leitung 105 und dem zweiten Abzweig 115 besteht.

### Bezugszeichen

- 101: Schmierstoffkreislauf
- 103: Ölsumpf
- 105: erste Leitung
- 107: Leitungsverzweigung
- 109: erste Pumpe
- 111: zweite Pumpe
- 113: erster Abzweig
- 115: zweiter Abzweig
- 117: Probenbehältnis
- 118: Leitungszusammenführung
- 119: Ölfilter
- 121: zweite Leitung
- 201: Schmierstoffkreislauf
- 203: Pumpe
- 205: Umschaltventil

## Patentansprüche

1. Anordnung von Mitteln, die einen Schmierstoffkreislauf (101) ausbilden;
mit mindestens einer Schmierstoffleitung (105), mindestens einer Leitungsverzweigung (107), welche die Schmierstoffleitung (105) in einen ersten Abzweig (113) und einen zweiten Abzweig (115) verzweigt, mindestens einem in den ersten Abzweig integrierten Schmierstoffspeicher (117) zum Entnehmen einer Schmierstoffprobe und mindestens ein Ventil, das in den ersten Abzweig (113) integriert ist, oder das in die Leitungsverzweigung (107) oder in eine Leitungszusammenführung (118) integriert
und als Umschaltventil ausgebildet ist; wobei
mindestens eine Leitungszusammenführung (118),den ersten Abzweig (113) und den zweiten Abzweig (115) zusammenführt; wobei
der erste Abzweig (113) sperr- und freigebbar ist; **dadurch gekennzeichnet, dass** das Ventil (205) temperaturgesteuert ist.

2. Anordnung von Mitteln, die einen Schmierstoffkreislauf (101) ausbilden;
mit mindestens einer Schmierstoffleitung (105), mindestens einer Leitungsverzweigung (107), welche die Schmierstoffleitung (105) in einen ersten Abzweig (113) und einen zweiten Abzweig (115) verzweigt und mindestens einem in den ersten Abzweig integrierten Schmierstoffspeicher (117) zum Entnehmen einer Schmierstoffprobe; wobei
mindestens eine Leitungszusammenführung (118),den ersten Abzweig (113) und den zweiten Abzweig (115) zusammenführt; wobei
der erste Abzweig (113) sperr- und freigebbar ist; **gekennzeichnet durch** mindestens einen Schmierstofffilter (119); wobei
der Schmierstofffilter (119) bezüglich des durch den Schmierstoffkreislauf (101) verlaufenden Schmierstoffflusses hinter der Leitungszusammenführung (118) angeordnet ist.

3. Anordnung nach Anspruch 1; **gekennzeichnet durch**
mindestens ein in den zweiten Abzweig (115) integriertes Ventil.

4. Anordnung nach einem der vorhergehenden Ansprüche; **gekennzeichnet durch** eine Pumpe (203), die bezüglich eines durch den Schmierstoffkreislauf (101) verlaufenden Schmierstoffflusses vor der Leitungsverzweigung (107) oder hinter der Leitungszusammenführung (118) angeordnet ist.

5. Anordnung nach Anspruch 2; **gekennzeichnet, durch**
eine erste, in den ersten Abzweig (113) integrierte Pumpe (109) und eine zweite, in den zweiten Abzweig (115) integrierte Pumpe (111).

6. Anordnung nach einem der vorhergehenden Ansprüche; **gekennzeichnet durch** mindestens ein in den ersten Abzweig (113) integriertes Rückschlagventil; wobei das Rückschlagventil bezüglich eines durch den ersten Abzweig (113) verlaufenden Schmierstoffflusses zwischen der Leitungsverzweigung (107) und dem Schmierstoffspeicher (117) angeordnet ist; wobei
das Rückschlagventil in Richtung des Schmierstoffspeichers (117) durchlässig ist und in Richtung der Leitungsverzweigung (107) sperrt.

7. Verfahren zur Bereithaltung einer validen Schmierstoffprobe unter Verwendung einer Anordnung nach einem der vorhergehenden Ansprüche
**dadurch gekennzeichnet, dass** ein oder mehrere physikalische Größen des Schmierstoffs gemessen werden; wobei das in den ersten Abzweig (113) integrierte Ventil, das als Umschaltventil ausgebildete Ventil (205), das in den zweiten Abzweig integrierte Ventil, die erste Pumpe (109) und/oder die zweite Pumpe (111) in Abhängigkeit der gemessenen physikalischen Größen gesteuert werden.

8. Anordnung nach einem der Ansprüche 1 bis 6; **gekennzeichnet durch** mindestens ein Steuergerät, das ausgebildet ist, ein Verfahren nach Anspruch 7 auszuführen.

## Claims

1. Arrangement of means which form a lubricant circuit (101);
having at least one lubricant line (105), at least one line branching point (107) which divides the lubricant line (105) into a first branch (113) and a second branch (115), at least one lubricant store (117) which is integrated into the first branch and serves for extraction of a lubricant sample, and at least one valve which is integrated into the first branch (113), or which is integrated into the line branching point (107) or into a line merging point (118) and is in the form of a switching valve; wherein
at least one line merging point (118) brings together the first branch (113) and the second branch (115); wherein the first branch (113) is able to be closed off and opened up; **characterized in that** the valve (205) is temperature-controlled.

2. Arrangement of means which form a lubricant circuit (101); having at least one lubricant line (105), at least one line branching point (107) which divides the lubricant line (105) into a first branch (113) and a second branch (115), and at least one lubricant store (117) which is integrated into the first branch and serves for extraction of a lubricant sample;
wherein
at least one line merging point (118) brings together the first branch (113) and the second branch (115); wherein the first branch (113) is able to be closed off and opened up; **characterized by**
at least one lubricant filter (119); wherein
the lubricant filter (119) is arranged behind the line merging point (118) in relation to the lubricant flow running through the lubricant circuit (101).

3. Arrangement according to Claim 1; **characterized by** at least one valve which is integrated into the second branch (115).

4. Arrangement according to one of the preceding claims; **characterized by**
a pump (203) which is arranged in front of the line branching point (107), or behind the line merging point (118), in relation to a lubricant flow running through the lubricant circuit (101).

5. Arrangement according to Claim 2; **characterized by** a first pump (109) which is integrated into the first branch (113), and a second pump (111) which is integrated into the second branch (115).

6. Arrangement according to one of the preceding claims; **characterized by**
at least one check valve which is integrated into the first branch (113); wherein
the check valve is arranged between the line branching point (107) and the lubricant store (117) in relation to a lubricant flow running through the first branch (113); wherein
the check valve is able to be passed through in the direction of the lubricant store (117) and is blocked in the direction of the line branching point (107).

7. Method for keeping a valid lubricant sample available using an arrangement according to one of the preceding claims,
**characterized in that**
one of more physical variables of the lubricant are measured; wherein
the valve integrated into the first branch (113), the valve (205) in the form of the switching valve, the valve integrated into the second branch, the first pump (109) and/or the second pump (111) are controlled according to the measured physical variables.

8. Arrangement according to one of Claims 1 to 6; **characterized by**
at least one control device which is configured to carry out a method according to Claim 7.

## Revendications

1. Agencement de moyens qui forment un circuit de lubrifiant (101) ;
comprenant au moins une conduite de lubrifiant (105), au moins une bifurcation de conduite (107), laquelle bifurque la conduite de lubrifiant (105) en une première conduite de dérivation (113) et une deuxième conduite de dérivation (115), au moins un réservoir de lubrifiant (117) intégré dans la première conduite de dérivation pour le prélèvement d'un échantillon de lubrifiant et au moins une soupape qui est intégrée dans la première conduite de dérivation (113), ou qui est intégrée dans la bifurcation de conduite (107) ou dans une jonction de conduites (118) et est réalisée sous forme de soupape de commutation ; dans lequel
au moins une jonction de conduites (118) réunit la première conduite de dérivation (113) et la deuxième conduite de dérivation (115) ; dans lequel la première conduite de dérivation (113) peut être bloquée et dégagée ; **caractérisé en ce que** la soupape (205) est commandée par température.

2. Agencement de moyens qui forment un circuit de lubrifiant (101) ;
comprenant au moins une conduite de lubrifiant (105), au moins une bifurcation de conduite (107), laquelle bifurque la conduite de lubrifiant (105) en une première conduite de dérivation (113) et une deuxième conduite de dérivation (115) et au moins un réservoir de lubrifiant (117) intégré dans la première conduite de dérivation pour le prélèvement d'un échantillon de lubrifiant ; dans lequel
au moins une jonction de conduites (118) réunit la première conduite de dérivation (113) et la deuxième conduite de dérivation (115) ; dans lequel la première conduite de dérivation (113) peut être bloquée et dégagée ; **caractérisé par**
au moins un filtre à lubrifiant (119) ; dans lequel le filtre à lubrifiant (119) est agencé derrière la jonction de conduites (118) par rapport au flux de lubrifiant s'étendant à travers le circuit de lubrifiant (101) .

3. Agencement selon la revendication 1 ; **caractérisé par**
au moins une soupape intégrée dans la deuxième conduite de dérivation (115).

4. Agencement selon l'une des revendications précédentes ; **caractérisé par**
une pompe (203) qui est agencée devant la bifurcation de conduite (107) ou derrière la jonction de conduites (118) par rapport à un flux de lubrifiant s'étendant à travers le circuit de lubrifiant (101).

5. Agencement selon la revendication 2 ; **caractérisé par**
une première pompe (109) intégrée dans la première conduite de dérivation (113) et une deuxième pompe (111) intégrée dans la deuxième conduite de dérivation (115).

6. Agencement selon l'une des revendications précédentes ; **caractérisé par**
au moins une soupape anti-retour intégrée dans la première conduite de dérivation (113) ; dans lequel la soupape anti-retour est agencée entre la bifurcation de conduite (107) et le réservoir de lubrifiant (117) par rapport à un flux de lubrifiant s'étendant à travers la première conduite de dérivation (113) ; dans lequel la soupape anti-retour peut être traversée en direction du réservoir de lubrifiant (117) et bloque en direction de la bifurcation de conduite (107).

7. Procédé de mise à disposition d'un échantillon de lubrifiant valide à l'aide d'un agencement selon l'une des revendications précédentes,
**caractérisé en ce**
**qu'**une ou plusieurs grandeurs physiques du lubrifiant sont mesurées ;
la soupape intégrée dans la première conduite de dérivation (113), la soupape (205) réalisée sous forme de soupape de commutation, la soupape intégrée dans la deuxième conduite de dérivation, la première pompe (109) et/ou la deuxième pompe (111) étant commandées en fonction des grandeurs physiques mesurées.

8. Agencement selon l'une des revendications 1 à 6 ; **caractérisé par**
au moins un appareil de commande qui est réalisé pour mettre en œuvre un procédé selon la revendication 7.
